# EUROPEAN PATENT SPECIFICATION

(11) **EP 1 591 109 B1**
(45) Date of publication and mention of the grant of the patent: **02.07.2008**
(21) Application number: 04010333.5
(22) Date of filing: 30.04.2004
(51) Int. Cl.: A61K 9/50, A61K 45/08

(54) **Formulation comprising histone deacetylase inhibitor exhibiting biphasic release**
Histondeacetylase-Inhibitor enthaltende Formulierung zur zweiphasigen Freisetzung
Formulation à libération biphasique comprenant un inhibiteur d'histone déacétylase

(43) Date of publication of application: 02.11.2005
(73) Proprietor: TopoTarget Germany AG, 60596 Frankfurt am Main (DE); Desitin Arzneimittel GmbH, D-22335 Hamburg (DE)
(72) Inventor: Franke, Hanshermann, Dr., 22889 Tangstedt (DE); Lennartz, Peter, Dr., 20253 Hamburg (DE); Maurer, Alexander, B., Dr., 60322 Frankfurt am Main (DE); Hentsch, Bernd, Dr., 60322 Frankfurt am Main (DE); Hövelmann, Sascha, Dr., 60386 Frankfurt am Main (DE); Martin, Elke, Dr., 76187 Karlsruhe (DE)
(74) Representative: Keller, Günter

(56) References cited:
- EP-A- 0 430 287
- WO-A-94/27587
- QIU Y ET AL: "ONCE-A-DAY CONTROLLED-RELEASE DOSAGE FORM OF DIVALPROEX SODIUM I: FORMULATION DESIGN AND IN VITRO/IN VIVO INVESTIGATIONS" JOURNAL OF PHARMACEUTICAL SCIENCES, AMERICAN PHARMACEUTICAL ASSOCIATION. WASHINGTON, US, vol. 92, no. 6, June 2003 (2003-06), pages 1166-1173, XP001161543 ISSN: 0022-3549

## Description

The present invention relates to a novel orally available galenics formulation of Valproic Acid or derivatives thereof exhibiting a specific bi-phasic pharmacokinetic profile optimized for maximum inhibition of histone deacetyiases in a therapeutic setting. This specific galenics formulation is designed for the treatment of malignant diseases and diseases associated with hypoacetylation of histones or in which induction of hyperacetylation has a beneficial effect, e.g., by induction of differentiation and/or apoptosis. Due to the bi-phasic release pattern the resulting pharmacokinetic profile is able to inhibit HDAC target enzymes most efficiently and to subsequently induce histone hyperacetylation in a rapid as well as a long-lasting fashion. This profile secures the efficient modulation of a desired target gene expression profile which contributes to the therapeutic benefit.

### BACKGROUND OF THE INVENTION

### Chromatin Regulation and Diseases

Local remodeling of chromatin is a key step in the transcriptional activation of genes. Dynamic changes in the nucleosomal packaging of DNA must occur to allow transcriptional proteins to make contact with the DNA template. One of the most important mechanisms influencing chromatin remodeling and gene transcription are the posttranslational modifications of histones and other cellular proteins by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). In the case of histone hyperacetylation, changes in electrostatic attraction for DNA and steric hindrance introduced by the hydrophobic acetyl group leads to destabilisation of the interaction of histones with DNA. As a result, acetylation of histones disrupts nucleosomes and allows the DNA to become accessible to the transcriptional machinery. Removal of the acetyl groups allows the histones to bind more tightly to DNA and to adjacent nucleosomes, and thus, to maintain a transcriptionally repressed chromatin structure. Acetylation is mediated by a series of enzymes with histone acetyltransferase (HAT) activity. Conversely, acetyl groups are removed by specific histone deacetylase (HDAC) enzymes. Disruption of these mechanisms gives rise to transcriptional misregulation and may contribute to a variety of human diseases, including autoimmune, inflammatory or hyperproliferative disorders including tumorigenic transformation and tumor progression.

Additionally, other molecules such as transcription factors alter their activity and stability depending on their acetylation status. E.g. PML-RAR, the fusion protein associated with acute promyelocytic leukemia (APL) inhibits p53 through mediating deacetylation and degradation of p53, thus allowing APL blasts to evade p53 dependent cancer surveillance pathways. Expression of PML-RAR in hematopoietic precursor cells results in repression of p53 mediated transcriptional activation, and protection from p53-dependent apoptosis triggered by genotoxic stresses (X-rays, oxidative stress). However, the function of p53 is reinstalled in the presence of HDAC inhibitors implicating active recruitment of HDAC to p53 by PML-RAR as the mechanism underlying p53 inhibition (Insinga et al., February 2004, EMBO Journal, 1-11). Therefore, acetylation of proteins distinct from histones, such as acetylation of p53, plays a crucial role in the anti-tumor activity of HDAC inhibitors.

### Nuclear Receptors and Histone Deacetylases

Nuclear hormone receptors are ligand-dependent transcription factors that control development and homeostasis through both positive and negative control of gene expression. Defects in these regulatory processes underlie the causes of many diseases and play an important role in the development of cancer. Many nuclear receptors, including T3R, RAR and PPAR, can interact with corepressors, such as N-CoR and SMRT, in the absence of ligand and thereby inhibit transcription. Furthermore, N-CoR has also been reported to interact with antagonist-occupied progesterone and estrogen receptors. Most interestingly, N-CoR and SMRT have been shown to exist in large protein complexes, which also contain mSin3 proteins and histone deacetylases (Pazin and Kadonaga, 1997; Cell 89, 325-8). Thus, the ligand-induced switch of nuclear receptors from repression to activation reflects the exchange of corepressor and coactivator complexes with antagonistic enzymatic activities.

### Gene Regulation by Nuclear Receptors

Such corepressor complexes which contain HDAC activity, not only mediate repression by nuclear receptors, but also interact with additional transcription factors including Mad-1, BCL-6, and ETO. Many of these proteins play key roles in disorders of cell proliferation and differentiation (Pazin and Kadonaga, 1997, Cell 89, 325-8; Huynh and Bardwell, 1998, Oncogene 17, 2473-84; Wang, J. et al., 1998, Proc Natl Acad Sci U S A 95, 10860-5). T3R for example was originally identified on the basis of its homology with the viral oncogene v-erbA, which in contrast to the wild type receptor does not bind ligand and functions as a constitutive repressor of transcription. Furthermore, mutations in RARs have been associated with a number of human cancers, particularly acute promyelocytic leukemia (APL) and hepatocellular carcinoma. In APL patients RAR fusion proteins resulting from chromosomal translocations involve either the promyelocytic leukemia protein (PML) or the promyelocytic zinc finger protein (PLZF). Although both fusion proteins can interact with components of the corepressor complex, the addition of retinoic acid dismisses the corepressor complex from PML-RAR, whereas PLZF-RAR interacts constitutively. These findings provide an explanation why PML-RAR APL patients achieve complete remission following retinoic acid treatment whereas PLZF-RAR APL patients respond very poorly (Grignani et al., 1998, Nature 391, 815-8; Guidez et al., 1998, Blood 91, 2634-42; He et al., 1998, Nat Genet 18, 126-35; Lin et al., 1998, Nature 391, 811-4).

Recently, a PML-RAR patient who had experienced multiple relapses after treatment with retinoic acid has been treated with the HDAC inhibitor phenylbutyrate, resulting in complete remission of the leukemia (Warrell et al., 1998, J. Natl. Cancer Inst. 90, 1621-1625).

### The Protein Family of Histone Deacetylases

The recruitment of histone acetyltranferases (HATs) and histone deacetylases (HDACs) is considered as a key element in the dynamic regulation of many genes playing important roles in cellular proliferation and differentiation. Hyperacetylation of the N-terminal tails of histones H3 and H4 correlates with gene activation whereas deacetylation can mediate transcriptional repression. Consequently, many diseases have been linked to changes in gene expression caused by mutations affecting transcription factors. Aberrant repression by leukemia fusion proteins such as PML-RAR, PLZF-RAR, AML-ETO, and Stat5-RAR serves as a prototypical example in this regard. In all of these cases, chromosomal translocations convert transcriptional activators into repressors, which constitutively repress target genes important for hematopoietic differentiation via recruitment of HDACs. It is plausible that similar events could also contribute to pathogenesis in many other types of cancer. There is growing evidence that the same holds true also for autoimmune, inflammatory or hyperproliferative disorders.

Mammalian histone deacetylases can be divided into three subclasses (Gray and Ekström, 2001). HDACs 1, 2, 3, and 8 which are homologues of the yeast RPD3 protein constitute class I. HDACs 4, 5, 6, 7, 9, and 10 are related to the yeast Hda 1 protein and form class II. Recently, several mammalian homologues of the yeast Sir2 protein have been identified forming a third class of deacetylases which are NAD dependent. Furthermore, HDAC11 has been classified as a class I histone deacetylase with structural features of a class II HDAC. All of these HDACs appear to exist in the cell as subunits of a plethora of multiprotein complexes. In particular, class I and II HDACs have been shown to interact with transcriptional corepressors mSin3, N-CoR and SMRT which serve as bridging factors required for the recruitment of HDACs to transcription factors.

### Therapy with HDAC Inhibitors

Additional clinical investigations have recently been initiated to exploit the systemic clinical treatment of cancer patients with the principle of HDAC inhibition. By now, a clinical phase II trial with the closely related butyric acid derivative Pivanex (Titan Pharmaceuticals) as a monotherapy has been completed demonstrating activity in stage III/IV non-small cell lung cancer (Keer et al., 2002, ASCO, Abstract No. 1253). More HDAC inhibitors have been identified, with NVP-LAQ824 (Novartis) and SAHA (Aton Pharma Inc.) being members of the structural class of hydroxamic acids tested in phase II clinical trials (Marks et al., 2001, Nature Reviews Cancer 1, 194-202). Another class comprises cyclic tetrapeptides, such as depsipeptide (FR901228 - Fujisawa) used successfully in a phase II trial for the treatment of T-cell lymphomas (Piekarz et al., 2001, Blood 98, 2865-8). Furthermore, MS-27-275 (Mitsui Pharmaceuticals), a compound related to the class of benzamides, is now being tested in a phase I trial treating patients with hematological malignancies.

### Valproic Acid

Valproic acid (VPA; 2-propyl-pentanoic acid) has multiple biological activities which depend on different molecular mechanisms of action:
- VPA is an antiepileptic drug.
- VPA is teratogenic. When used as an antiepileptic drug during pregnancy, VPA can induce birth defects (neural tube closure defects and other malformations) in a few percent of born children. In mice, VPA is teratogenic in the majority of mouse embryos when properly dosed.
- VPA activates a nuclear hormone receptor (PPARδ). Several additional transcription factors are also derepressed but some factors are not significantly derepressed (glucocorticoid receptor, PPARa).
- VPA occasionally causes hepatotoxicity, which may depend on poorly metabolized esters with coenzyme A.
- VPA is an inhibitor of HDACs.

The use of VPA derivatives allowed to determine that the different activities are mediated by different molecular mechanisms of action. Teratogenicity and antiepileptic activity follow different modes of action because compounds could be isolated which are either preferentially teratogenic or preferentially antiepileptic (Nau et al., 1991, Pharmacol. Toxicol. 69, 310-321). Activation of PPARδ was found to be strictly correlated with teratogenicity (Lampen et al., 1999, Toxicol. Appl. Pharmacol. 160, 238-249) suggesting that, both, PPARδ activation and teratogenicity require the same molecular activity of VPA. Also, differentiation of F9 cells strictly correlated with PPARδ activation and teratogenicity as suggested by Lampen et al., 1999, and documented by the analysis of differentiation markers (Werling et al., 2001, Mol. Pharmacol. 59, 1269-1276). It was shown, that PPARδ activation is caused by the HDAC inhibitory activity of VPA and its derivatives (WO 02/07722 A2; WO 03/024442 A2). Furthermore, it was shown that the established HDAC inhibitor TSA activates PPARδ and induces the same type of F9 cell differentiation as VPA. From these results it can be concluded that not only activation of PPARδ but also induction of F9 cell differentiation and teratogenicity of VPA or VPA derivatives are caused by HDAC inhibition.

Antiepileptic and sedating activities follow different structure activity relationships and thus obviously depend on a primary VPA activity distinct from HDAC inhibition. The mechanism of hepatotoxicity is poorly understood and it is unknown whether it is associated with formation of the VPA-CoA ester. HDAC inhibition, however, appears not to require CoA ester formation.

### Valproic Acid as inhibitor of histone deaceylases

VPA has been developed as a drug used for the treatment of epilepsia. Accordingly, VPA is used systemically, orally, or intravenously, to allow the drug to pass the blood brain barrier to reach the epileptic target regions in the brain tissue in order to fulfill its antiepileptic mission. Moreover, VPA has been shown to possess beneficial effects when used for the treatment of many different types of human cancers as a single agent or in combination with a whole variety of other anti-tumor therapies which are individually based on strikingly different modes of action by inhibiting specific sets of enzymes having HDAC activity and thereby inducing differentiation and/or apoptosis (WO 02/07722 A2, EP 1170008; WO 03/024442 A2, EP 1293205 A1). For the treatment or prevention of malignant diseases autoimmune diseases or other inflammatory or hyperproliferative disorders, VPA may also be administered systemically, orally, or intravenously. Furthermore, it was shown, that VPA permeates human skin effectively and therefore can be administered topically on skin exhibiting beneficial effects when used for the topical treatment or prevention of autoimmune, inflammatory or hyperproliferative human skin diseases, e.g., psoriasis and human skin cancer (EP application No. 03014278.0).

### A tailor-made formulation of VPA for cancer treatment

For oral administration, VPA has been developed in "slow release" as well as "fast release" application formulations. However, using a "slow release" application formulation will result in a slow increase of VPA levels in the blood over a long period of time without efficiently reaching VPA plasma concentrations required for the inhibition of enzymes having histone deacetylase activity. Furthermore, cellular compensatory counter-mechanisms might be induced during this period of slowly increasing VPA levels before effective serum doses are reached rendering VPA less effective in inhibiting enzymes having histone deacetylase activity. A formulation based exclusively on a "fast release" formulation of VPA on the other hand will lead to a high initial level of VPA in the blood, resulting only in a short period of effective HDAC inhibition.

Surprisingly, the inventors could demonstrate that not only the absolute concentration of VPA in the serum, but also the duration of effective levels of VPA during treatment are crucial for maximum inhibition of histone deacetylase activity. The desired and most beneficial pharmacokinetic profile can not be obtained by the use of acquainted and well established galenics formulations.

Therefore, considering the shortcomings of established formulations, the present invention relates to a pharmaceutical formulation as defined in claim 1. Preferably, the formulation is an orally available formulation.

The term "release" as used herein refers to the release of the histone deacetylase inhibitor from the pharmaceutical formulation.

As used herein, the term "release profile" refers to the release of the histone deacetylase inhibitor over a given period of time. Methods to determine the release profile of a pharmaceutical formulation *in vitro* are known to those skilled in the art. A preferred method in accordance with this invention is U.S. Pharmacopeia (USP) 24, method 724, apparatus 2, in 900ml buffer pH 6.8 USP at 100 rpm.

A "bi-phasic" release profile shows a first phase of fast release (immediate release) followed by a second phase of slow release (sustained release). Preferably, 10-60% of the histone deacetylase inhibitor in the formulation is released within 30 minutes, and 50-100% of the histone deacetylase inhibitor in the formulation is released within 6 hours. More preferably, 20-50% of the histone deacetylase inhibitor in the formulation is released within 30 minutes, and 60-100% of the histone deacetylase inhibitor in the formulation is released within 6 hours.

As used herein, the term "histone deacetylase inhibitor" denotes a substance that is capable of inhibiting the histone deacetylase acitivity of an enzyme having histone deacetylase acitivity.

The inhibitory acitivity of a histone deacetylase inhibitor can be determined in an in vitro assay as described in Example 1 of this application. The IC₅₀ value can be taken as a measure for the inhibitory acitivity of a histone deacetylase inhibitor. A low IC₅₀ value indicates a high inhibitory activity; a high IC₅₀ value indicates a low inhibitory activity. The histone deacetylase inhibitors used in accordance with this invention preferably have an IC₅₀ value of less than 1 mM, more preferably of less than 500 µM with respect to at least one histone deacetylase.

According to a preferred embodiment, the histone deacetylase inhibitor is capable of inhibiting preferentially a subset of histone deacetylases or selected deacetylases. The term "inhibiting preferentially" as used herein refers to a situation where a first group of histone deacetylases are inhibited more strongly than a second group of histone deacetylases by a given histone deacetylase inhibitor. Usually, the histone deacetylase inhibitor inhibiting preferentially a first group of histone deacetylases has an IC₅₀ value of less than 800 µM, preferably of less than 500 µM with respect to the histone deacetylases of said first group. The IC₅₀ value with respect to histone deacetylases of the second group is usually greater than 800 µM, preferably greater than 1 mM.

In a first specific embodiment, the histone deacetylase inhibitor is capable of inhibiting preferentially class I histone deacetylases. According to this first embodiment, class I histone deacetylases are inhibited more strongly than class II histone deacetylases. In this first embodiment, the histone deacetylase inhibitor usually has IC₅₀ values of less than 800 µM, preferably of less than 500 µM with respect to the histone deacetylase enzymes HDAC 1, 2, 3 and 8. In addition, the histone deacetylase inhibitor usually has IC₅₀ values of greater than 800 µM, preferably of greater than 1 mM with respect to the class II enzymes HDAC 4, 5, 6, 7, 9 and 10.

Histone deacetylase inhibitors in accordance with this invention are valproic acid and pharmaceutically acceptable salts of valproic acid. Most preferred are valproic acid and pharmaceutically acceptable salts thereof such as sodium valproate.

### Galenics formulation, dosing and pharmacokinetic profile of Valproate

The optimum serum profile for b.i.d. oral dosing at steady state is characterized by allowing a rapid increase of the VPA-serum concentration to levels between 90 to 200 µg/ml within 30 minutes and more preferentially between 110 to 180 µg/ml. This serum concentration level remains constant for 8 to 10 hours and then decreases below 110 µg/ml. However, the serum concentration level of VPA stays permanently above 80 µg/ml during treatment more preferentially above 100 µg/ml. The indicated periods of time refer to (start with) the time of oral administration.

In the case of controlled release formulations for oral administration multiple unit dosage forms are superior to single unit dosage forms. The release of the active ingredient is independent from the degree of filling of the stomach and results in similar release profiles even in different patients. Furthermore the phenomenon of dose dumping (J Butler et al., Pharm. Technol. 1998, 122-138) can be avoided.

Various pharmaceutical compositions for the administration of VPA and salts thereof are commonly available including parenterals, oral solutions, coated tablets with resistance to gastric fluids, slow releasing tablets and minitablets. Because of the liquid nature of VPA as well as the hygroscopic nature of sodium valproate, the formulation of multiple unit dosage forms is technologically challenging.

The desired VPA serum concentration levels as described above can be obtained by a combination of fast and slow in vitro release pattern that cannot be obtained by existing formulations to effectively inhibit the HDAC target enzymes. For the treatment or prevention of, e.g, cancer or other hyperproliferative or inflammatory disorders by inhibition of histone deacetylases, there is a need for a novel pharmaceutical formulation for oral administration which provides the desired serum concentration profile of VPA.

These requirements are preferably met by a pharmaceutical composition with a bi-phasic release pattern of histone deacetylase inhibitors, e.g., sodium valproate. The pharmaceutical formulation according to the invention therefore comprises a fast releasing component and a slow releasing component, usually in a predefined proportion. In a particular embodiment, the pharmaceutical formulation consists essentially of a fast releasing component and a slow releasing component in a predefined proportion.

The ratio of fast releasing component to slow releasing component is preferably between 1:0.5 and 1:4, more preferably between 1:1 and 1:3.

The pharmaceutical formulation preferably shows an in vitro release of 20 to 50 % within 30 minutes, of 25 to 65 % within 2 hours, of 55 to 85 % within 4 hours and 70 to 100 % within 6 hours (USP 24, method 724, app. 2, in 900ml buffer pH 6.8 USP at 100 rpm). The water uptake of the combination of both components is usually below 5 % within 24 hours when exposed to 40 % relative humidity at 25 °C.

The fast releasing component preferably shows an in vitro release of at least 90 % of histone deacetylase inhibitor (e.g. sodium valproate) within 15 minutes (USP 24, method 724, app. 2, in 900ml buffer pH 6.8 USP at 100 rpm). The water uptake of the component is regularly below 5 % within 24 hours when exposed to 40 % relative humidity at 25 °C.

The slow releasing component preferably shows an in vitro release of 0 to 30 % within 1 hour, of 20 to 60 % within 4 hours, and of 55 to 95 % within 6 hours (USP 24, method 724, app. 2, in 900 ml buffer pH 6.8 USP at 100 rpm). The water uptake of the component is generally below 5 % within 24 hours when exposed to 40 % relative humidity at 25 °C.

The slow releasing component usually has a content of histone deacetylase inhibitors (e.g. sodium valproate) of 50 to 96 % by weight, preferably of 70 to 95 %. The fast releasing component usually has a content of histone deacetylase inhibitors (e.g. sodium valproate) of 50 to 96 % by weight, preferably of 70 to 95 %.

Since a high amount of drug should be administered, a multiparticulate formulation is preferred. Therefore, the pharmaceutical formulation of the invention is in one embodiment a multiple unit dosage form comprising compartments. The term "compartment" denotes a particle containing a histone deacetylase inhibitor. The particle may have one or more coatings. The histone deacetylase inhibitor contained in the particle is preferably separated from the environment by said one or more coatings. Preferably, the compartments are coated microtablets. The compartments may be of different shape, preferably they are shaped spherically or bi-convexly. The maximum size (e.g., diameter) of the single compartments is usually 3 mm, preferably the size of the single compartments is 0.5 to 2.5 mm.

The fast releasing component comprises compartments, preferably the fast releasing component consists essentially of compartments. The slow releasing component comprises compartments, preferably the slow releasing component consists essentially of compartments. In accordance with the invention, the fast releasing component and the slow releasing component comprise compartments, preferably the fast releasing component and the slow releasing component consist essentially of compartments.

The single compartments of the fast releasing component differ from those of the slow releasing component.

The single compartments of the fast releasing component show very fast release of histone deacetylase inhibitors (e.g. sodium valproate) after oral administration. They can be prepared by commonly known granulation, pelletizing or tabletting techniques.

In comparison to the pure substance superior handling properties are achieved by a reduced hygroscopicity that is obtained by using suitable excipients and preparation processes. For example, the components can be coated with a suitable polymer in order to achieve the reduced hygroscopicity.

The single compartments of the slow releasing component show slow release of histone deacetylase inhibitors (e.g. sodium valproate) after oral administration. The maximum size of the compartments is usually 3 mm. They can be prepared by commonly known granulation, pelletizing or tabletting techniques.

As already mentioned, superior handling properties are achieved by reduced hygroscopicity when compared to the pure drug. The reduced hygroscopicity is established by using suitable excipients and preparation processes. For example, the components can be coated with a suitable polymer in order to achieve the reduced hygroscopicity and the slow release pattern.

The compartments may have a content of histone deacetylase inhibitor (e.g. sodium valproate) of 50 to 95 % by weight, preferably 60 to 85 %.

In one aspect of the invention, the compartments are coated minitablets. Usually, the coated minitablets of the fast releasing component differ from those of the slow releasing component in their coating.

In a specific aspect, the coated minitablets comprise at least one histone deacetylase inhibitor (e.g., sodium valproate), a lubricant, a polymer and a glidant. Preferably, the coated minitablets consist essentially of these constituents. The lubricant is preferably magnesium stearate, calcium stearate and/or stearic acid. Suitable glidants include silicium dioxide, methylated silicium dioxide and/or talc. The polymer may be ammonio methacrylate copolymer, ethylcellulose and/or hypromellose.

In another aspect, the coating of the coated minitablets of the fast release component comprises at least one polymer and at least one suitable plasticizer. The polymer is preferably aminoalkyl methacrylate copolymer, polyvinyl alcohol and/or hypromellose. Suitable plasticizers include Triacetin, Dibutyl sebacate, Triethyl citrate, Polyethylene glycol. Additional plasticizers can be reviewed in the literature (e.g., Lexikon der Hilfsstoffe, H.P. Fiedler, Editio Cantor Verlag Aulendorf, 4. Auflage 1998).

In yet another aspect, the coating of the coated minitablets of the slow release component comprises at least one polymer and at least one suitable plasticizer. Suitable polymers are ammonio methacrylate copolymer and/or ethylcellulose.

Both components may be present in a predefined proportion in capsules or containers for single dose administration. The content of histone deacetylase inhibitors (e.g. sodium valproate) in a capsule or container for single dose administration may range from 0.1 to 3 g, preferably from 0.2 to 1.5 g.

Container for single dose administration can be sachets or pouches. It may consist of an aluminium foil with a minimum thickness of 9 µm or alternatively coated paper or other materials with comparable characteristics in order to provide a sufficient barrier against humidity.

The optimum amount of a histone deacetylase inhibitor (e.g. sodium valproate) for treatment is individually achieved by administration of the required amount of capsules or containers for single dose administration at each dosing interval. The optimum amount of a histone deacetylase inhibitor (e.g. sodium valproate) for treatment depends on the weight of the patient.

The invention further relates to a method for the preparation of a pharmaceutical formulation exhibiting a bi-phasic release profile, comprising combining a fast releasing component containing a histone deacetylase inhibitor with a slow releasing component containing a histone deacetylase inhibitor such that a multiple unit dosage form comprising compartments is obtained. The various embodiments described herein with respect to the pharmaceutical formulation of the invention apply to this method *mutatis mutandis.*

Preferably, the single drug containing compartments (e.g. coated minitablets) are prepared by granulation, extrusion, hot melt, pelletizing, tabletting and coating techniques.

Both components may be mixed in a predefined proportion and filled in capsules or containers for single dose administration. Alternatively they may be filled successively in capsules or containers for single dose administration without mixing them beforehand. The content of sodium valproate in a capsule or container for single dose administration may range from 0.1 to 3 g, preferably from 0.2 to 1.5 g.

The invention further relates to the use of a pharmaceutical formulation described herein for the manufacture of a medicament for the treatment or prevention of estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemias, lymphomas and/or other blood cell cancers, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity.

In another aspect, the invention relates to the use of valproic acid or a pharmaceutically acceptable salt thereof for the manufacture of a medicament for the treatment or prevention of one or more of these disorders, wherein the medicament is a pharmaceutical formulation as defined in claim 1. The various embodiments described herein with respect to the pharmaceutical formulation of the invention apply to this use *mutatis mutandis.*

Further described herein is a method of treating one or more of the disorders listed above, comprising administering to a patient in need thereof an effective amount of a pharmaceutical formulation described herein before. The administration of the effective amount of the pharmaceutical formulation is suitable to ameliorate the condition of the patient to be treated. The preferred embodiment described herein with respect to the pharmaceutical formulation of the invention apply to this method of treatment *mutatis mutandis.*

The present invention provides a pharmaceutical formulation having a bi-phasic pharmacokinetic release profile for the effective inhibition of HDAC proteins. The formulation displays highly beneficial characteristics without enhancing negative side effects. Here, an initial fast release of the compound leads to a pharmaceutical relevant concentration inhibiting cellular HDAC activity shortly after the administration of the drug. The subsequent slow release of additional compound is able to maintain HDAC inhibition at serum levels slightly above the effective therapeutic dose for an extended period of time. This sustained constant concentration of the compound within the therapeutic range results in a prolonged effect of VPA on the target enzymes having histone deacetylase activity. This effect can be monitored by the analysis of surrogate markers such as histone hyperacetylation in peripheral blood of VPA treated patients. Importantly, VPA is known to preferentially inhibit HDAC class I (one) isoenzymes in contrast to its weaker inhibitory activity for HDAC class II (two) enzymes. This profile is highly wanted, as the inhibition of HDAC class II enzymes might be associated with cardiotoxic side effects (Zhang et al., Cell 2002, 110:479-488; Antos et al., JBC 2003, 278:28930-7). Thus, sustained VPA serum levels in pharmaceutically relevant concentrations lead to a prolonged inhibition of histone deacetylases - in particular of class I isoenzymes - minimizing cardiotoxic side effects.

This prolonged inhibition of enzymes exhibiting histone deacetylase activity in patients with malignant conditions and/or diseases based on aberrant recruitment of histone deacetylases such as hyperproliferative or inflammatory disorders by applying a pharmaceutical formulation of inhibitors of histone deacetylases described in this invention, clearly is a novel approach for optimizing the treatment or prevention strategies of patients suffering from such afflictions. Thus, the use of an orally available composition combining a fast and a slow release pharmacokinetic profile is regarded to be highly beneficial for the application of VPA or other inhibitors of histone deacetylases in the treatment or prevention of hyperproliferative disorders such as malignant tumor diseases or inflammatory diseases.

In another embodiment of this invention, other types of application of inhibitors of histone deacetylases are included, such as but not limited to intraveneous, intramuscular, subcutaneous, topical (including plasters), other oral, nasal, intraperitoneal or suppository based (abdomino-anal) applications which may allow to create the release pattern and serum concentration levels of inhibitors of histone deacetylases as described in this invention.

### FIGURES

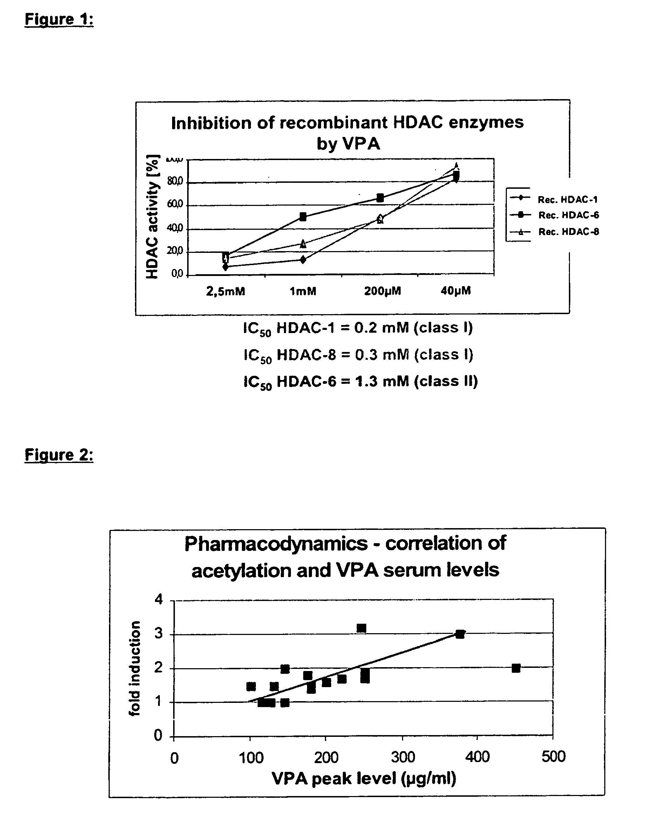

**Figure 1****:** *VPA inhibits the activity of recombinant HDAC enzymes*
   Figure 1 shows that VPA preferentially inhibits the tumor relevant class I HDAC enzymes (IC₅₀ of about 200 µM; exemplified for the class I enzyme HDAC 1) and is less active on class II HDAC enzymes (IC₅₀ of about 1.1 mM, exemplified for the class II enzyme HDAC 8). In respect to these data, it is important to note, that the pharmacokinetic data obtained in a Phase I clinical study revealed serum levels of VPA in cancer patients sufficient to successfully inhibit these relevant class I isoenzymes. In contrast, levels required for inhibition of class II HDAC enzymes can be avoided. This is a highly wanted profile since inhibition of class II enzymes is expected to cause cardiotoxicity (Zhang et al., Cell, 2002, 110:479-488; Antos et al., JBC, 2003, 278:28930-7).
**Figure 2****:** *Correlation between VPA serum levels and histone hyperacetylation*
   Figure 2 shows results from a clinical Phase I/II study using VPA intravenously with patients exhibiting advanced malignant diseases. Induction of histone hyperacetylation (presented as "fold induction") as a marker for the efficacy of VPA treatment was examined in peripheral blood cells collected from patients before and 6h, 24h as well as 48h after VPA treatment start. A clear correlation of VPA serum peak levels (presented in µg/ml) with the induction of histone hyperacetylation was observed.

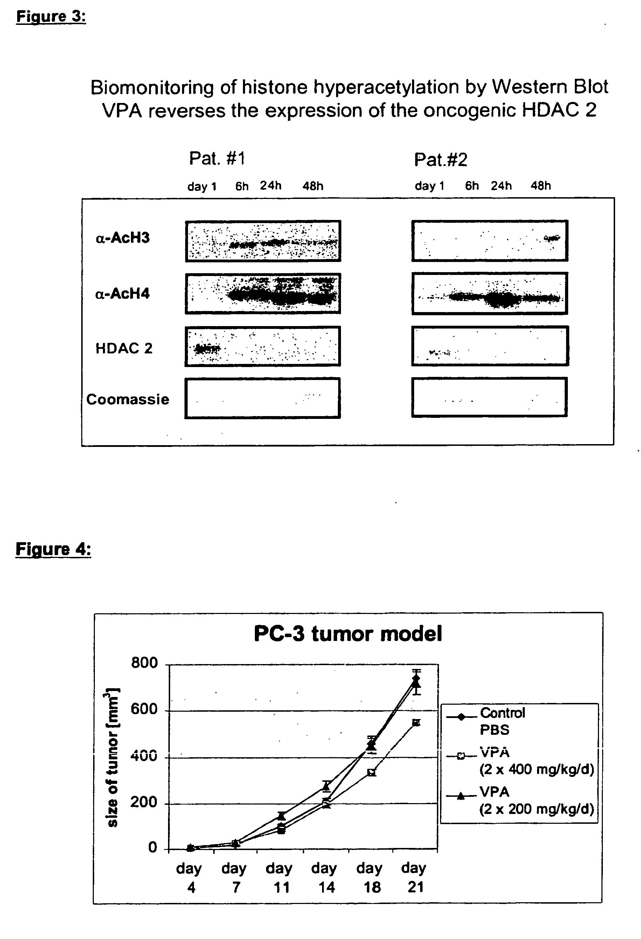

**Figure 3****:** *VPA induces histone hyperacetylation and regulation of marker genes in peripheral blood from patients from* a *phase I*/*II trial*
   Figure 3 displays a Western Blot analysis with peripheral blood cell lysates obtained from two patients (Pat. #1 and Pat. #2) exhibiting advanced malignant disease treated with VPA intravenously in the scope of a clinical Phase I/II study. Blood samples were taken before and 6h, 24h as well as 48h after treatment start. Histone H3 and H4 hyperacetylation and down regulation of the marker protein HDAC 2 could be detected in patients with serum levels above the therapeutic plasma concentration.
**Figure 4****:** *PC-3 mouse xenograft model*
   Figure 4 shows the results from a mouse PC-3 xenograft model. 24 athymic Nu/Nu^{-/-} mice were injected with 1x10⁶ PC3 prostate carcinoma cells in 100µl PBS into the right flank (8 animals per group). Tumors were allowed to grow for 4 days. Animals were treated with PBS (control), 2x200mg/kg/d or 2x400mg/kg/d, respectively, from day 5 until day 21. Tumor volumes were measured every 3-4 days. Here, again it became apparent that certain threshold doses have to be administered in order to have beneficial anti-tumor effects (tumor reduction by >25% when mice were treated with 400mg/kg/d twice daily, while no anti-tumoral effect could be detected in mice treated with 200mg/kg/d twice daily).

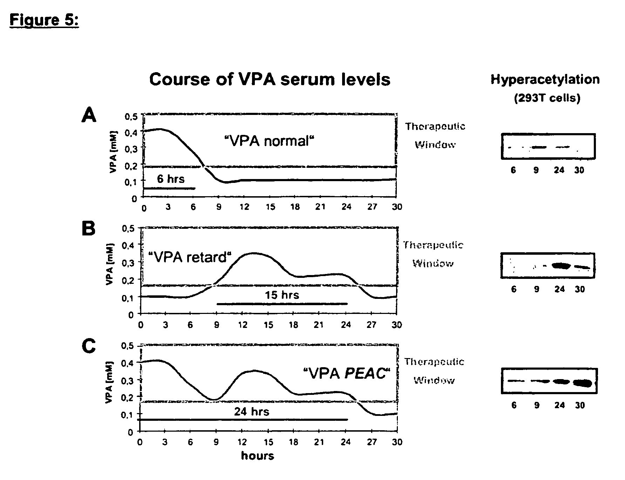

**Figure 5****:** *Histone hyperacetylation induced by various VPA formulations*
   Figure 5 exemplifies the proposed course of VPA serum levels in a "fast release" ('VPA "normal"' - A), a "slow release" ("VPA retard" - B), and a novel bi-phasic pharmacokinetic profile ("VPA ***PEAC***" C). Lysates of 293T cells treated with VPA for the indicated times (in hours) representative for either the "fast release" (A), the "slow release" (B), or the novel bi-phasic pharmacokinetic profile ***PEAC*** (C) were analysed in a Western Blot analysis using an anti-Histone H3 antibody. In comparison, the use of a "fast release" or a "slow release" formulation each on their own is less effective in respect to the degree of histone hyperacetylation induction as compared to the activity of the ***PEAC*** concept combining both release characteristics in the novel bi-phasic galenics formulation described herein.

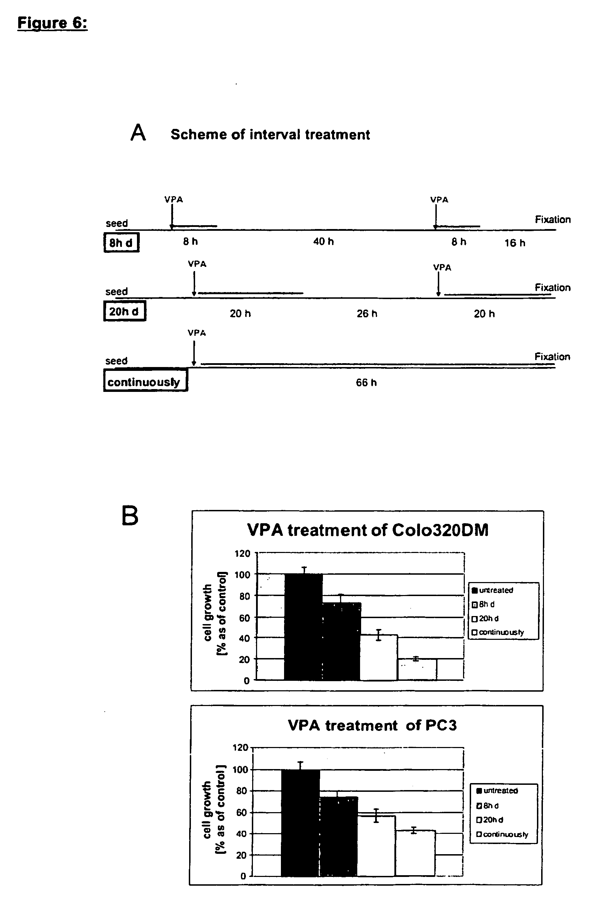

**Figure 6**: *Interval treatment of Colo320DM and PC-3 cell lines*
   Figure 6A depicts a VPA treatment schedule for Colo320DM and PC-3 cell lines. Cells were either treated with 1 mM VPA for 2 x 8 h with a 40h treatment free interval ("8h d") representative for a "fast release" VPA formulation, or treated for 20h twice with a 26h treatment free interval ("20h d") representative for a "slow release" formulation, or treated for 66h continuously representing the serum levels achievable using the novel bi-phasic compound release profile according to the ***PEAC*** concept ("continuously").
   Figure 6B shows results from SRB assays with Colo320DM and PC-3 cell lines treated according to the schedule described in Figure 6A. Whereas 2 x 8 hours ("8h d") exposure leads to only 26% (PC3) and 27% (Colo320DM) growth inhibition, 2 x 20 hours ("20h d") exposure increases growth inhibition to 43% in PC3 cells and 57% in Colo320DM cells. Maximum inhibition is seen at continuous exposure to VPA, representing the therapeutic serum levels that would be achieved using the bi-phasic release profile over an extended period of time, with 57% inhibition in PC3 and 80% in Colo320DM ("continuously").

### Table 1: VPA serum concentrations from phase I/II study with VPA intravenous administration

This table displays the VPA serum level concentration requirements in order to efficiently inhibit class I HDAC isoenzymes. At a total serum level of about 144.2 µg/ml, there is a free fraction (i.e., not serum protein bound) of VPA which is in the concentration range of the IC₅₀ of class I enzyme inhibition (about 0.2 mM). Neuronal side effects have been observed from total VPA plasma concentrations above 210 µg/ml (= 1.45mM). Based on these data, we have developed the novel galenics formulation of VPA described in this invention. This formulation secures an efficient inhibition of the most relevant HDAC class I target enzymes and subsequently induces histone hyperacetylation rapidly and long-lasting, whereas serum levels (especially of free VPA) as they would be required for the inhibition of class II HDAC enzymes are not reached. (MW: Molecular Weight)

### EXAMPLES

### Example 1

VPA, which acts as a preferential inhibitor of histone deacetylase class I enzymes (Figure 1), induces histone hyperacetylation in cellular systems as well as in peripheral blood cells of patients (Figure 3). The presented evidence for this relates also to the following patents: WO 02/07722 A2, EP 1170008; WO 03/024442 A2, EP 1293205 A1; EP application No. 03014278.0.

### Methods:

*in vitro HDAC assay for determination of IC₅₀ values:* The determination of histone deacetylase activity in recombinant HDAC proteins derived from expression in High5 insect cells is based on the specific deacetylation of an artificial substrate (Fluor de Lys, Biomol). The substrate turn over may be detected and quantified by fluorometry. By addition of a HDAC inhibitor the hydrolysis of the substrate is constrained resulting in a decreased fluorometric signal. IC₅₀ values may be calculated from dose-response curves. The assay is separated in two steps: in the first step the substrate (Fluor de Lys / Biomol KI-104) is hydrolysed by histone deacetylases. In step two HDAC activity is terminated and the fluorophore is activated by the addition of a developer (Developer / Biomol Kl-105). Recombinant proteins and the HDAC inhibitor are mixed with reaction buffer (Biomol Kl-143) to a total volume of 25µl per well of a 96 well plate. 25µl substrate (1:100 dilution in reaction buffer) per well are added to start the reaction. A negative control without histone deacetylase activity and a positive control without HDAC inhibitor are treated likewise. The reaction is stopped after 15-60min. by adding 50µl developer (1:20 dilution in reaction buffer). After another 15min. incubation time at room temperature the fluorescence signal is stable for 60min and may be detected by a fluorescence reader (excitation filter: 390nm, emission filter: 460nm). Recombinant histone deacetylases can be prepared and purified as described in Buggy et al., Cloning and characterization of a novel human histone deacetylase, HDAC8. Biochem J. 2000 Aug 15;350 Pt 1:199-205.

*Mouse Xenograft Model:* 24 athymic Nu/Nu^{-/-} (Harlan) mice were injected with 1x10⁶ PC3 prostate carcinoma cells in µl PBS into the right flank (8 animals per group). Tumors were allowed to grow for 4 days. Animals were treated with PBS (control), or VPA at 2x200mg/kg/d or 2x400mg/kg/d, respectively, from day 5 until day 21. Tumor volumes were measured every 3-4 days.

*Western blot:* Peripheral blood cells from patients treated with VPA intravenously in a clinical phase I/II trial were obtained before, 6h, 24h, and 48h after start of VPA treatment. Whole cell extracts were prepared by lysis of cells in RIPA buffer plus protease inhibitors for denaturing SDS gel electrophoresis on a 12% denaturing polyacrylamide gel. Acetylated histones H3 and H4 and marker protein HDAC 2 were detected by Western blot analysis using an anti-acetyl Histone H3 antibody (Upstate, #06-942), an anti-acetyl Histone H4 antibody (clone T25; patent application EP 02.021984.6), and an anti-HDAC 2 antibody (SCBT, SC-7899). As an equal loading control PVDV membranes were stained with Coomassie.

### Results:

In previous patent applications we have presented evidence that VPA can be used for the treatment of many different types of human cancers and other hyperproliferative or inflammatory disorders as a single agent or in combination with a whole variety of other anti-tumor therapies which are individually based on strikingly different modes of action (Patent applications: WO 02/07722 A2, EP 1170008; WO 03/024442 A2, EP 1293205 A1; EP application No. 03014278.0). Here, we show evidence that VPA acts as a preferential inhibitor of histone deacetylase class I enzymes (Figure 1) and can be used in patients to reach effective therapeutic serum concentrations inducing histone hyperacetylation and regulation of a target protein, HDAC 2 (Figure 3).

Figure 1 shows results from an *in vitro* assay examining the HDAC isoenzyme inhibitory specificity of VPA. Generating dose-response curves using various doses of VPA on recombinant proteins purified from High 5 insect cells, it became apparent that VPA preferentially inhibits HDAC class I enzymes as the IC₅₀ values for HDAC 1 und 8 (both class I) are 200 µM and 300 µM, respectively, while the IC₅₀ value for HDAC 6 (class II) is 1.1mM. These data are supported by results obtained from isolated human HDAC enzymes in immunoprecipitates (Göttlicher et al., EMBO J. (2001), 20:6969-78). Such immunoprecipition assays revealed, that the VPA inhibitory IC₅₀ values for class I HDAC enzymes (e.g. HDAC 1, 2, 3, and 8) range from approximately 100 µM to 400 µM and for class II enzymes (e.g. HDAC 5, 6, and 10) from 1100 to 2800 µM. This preferential inhibition of HDAC class I enzymes is a highly wanted profile as the inhibition of HDAC class II enzymes might be associated with cardiotoxic side effects (Zhang et al., Cell 2002, 110:479-488; Antos et al., JBC 2003, 278:28930-7).

In addition, in vivo data obtained in mouse PC-3 xenograft models show, that certain threshold doses have to be administered in order to achieve beneficial anti-tumor effects. As can be seen in Figure 4, PC-3 tumor volumes were reduced by >25% when mice were treated with 400 mg/kg/d VPA twice daily as compared to PBS control-treated tumors, while no anti-tumoral effect could be detected when mice were treated with 200 mg/kg/d VPA twice daily.

Table 1 displays VPA serum levels obtained in patients treated with VPA intravenously within a phase I/II trial showing that effective serum levels inhibiting HDAC enzymes can be reached in patients. Neuronal side effects have been observed from total VPA serum levels above 210 µg/ml (approximately 1.45 mM). Therefore, tolerable therapeutic serum concentrations will be far higher than the effective dose needed for HDAC class I inhibition (around 0.2 mM of free VPA, approximately 1.0 mM of total VPA) but still low enough to not inhibit HDAC class II enzymes, thereby avoiding cardiotoxic side effects.

**Table 1:**

| |
|---|
| IC50 for HDAC class I = 0.2 mM |
| |
| 144.2 µg/ml total VPA = 1.0 mM (MW_{VPA} 144.2) |
| 28.8 µg/ml free VPA = 0.2 mM (app. 20 % of total VPA) |
| |
| Neuronal side effects = > 210 µg/ml ≈ 1.45 mM |

Figures 2 and 3 present data from patients treated with VPA intravenously in a phase I/II trial. Induction of histone hyperacetylation as a marker for the efficacy of VPA treatment was examined in peripheral blood cells collected from patients before and 6h, 24h as well as 48h after VPA treatment start. A clear correlation of VPA serum peak levels with the induction of histone hyperacetylation was observed (Figure 2). Furthermore, histone H3 and H4 hyperacetylation and down regulation of the marker protein HDAC 2 could be detected in patients with serum levels above the therapeutic plasma concentration (Figure 3).

Thus, VPA is an isoenzyme specific inhibitor of histone deacetylases not only in cellular systems but also in a therapeutic setting for the treatment or prevention of patients with malignant tumor diseases or other hyperproliferative or inflammatory disorders.

### Example 2

Maximum HDAC inhibition by VPA requires both, an initial peak concentration followed by a prolonged, sustained concentration above the therapeutic level.

### Methods:

*Western blot:* 293T cells were seeded in 6-well plates and treated according to a scheme representing a "fast release" ("VPA normal"), a "slow release" ("VPA retard"), and a bi-phasic ("VPA *PEAC*") release pattern. The duration of exposure was calculated as 6 hours representing the "fast release" normal VPA formulation, 15 hours representing the retarded "slow release" formulation of VPA and 24 hours representing the bi-phasic release pattern of the *PEAC* formulation. Whole cell extracts were prepared by lysis of cells in RIPA buffer plus protease inhibitors for denaturing SDS gel electrophoresis on a 12% denaturing polyacrylamide gel. Acetylated histones H3 were detected by Western blot analysis using an anti-acetylated H3 antibody (Upstate, #06-942).

*SRB proliferation assay:* The reduction in cellular biomass was measured by SRB-assay. For this assay cells were seeded in 96 well culture dishes at densities between 3000 and 8000 cells per well. After recovery of 24 hours, cells were cultured for 72 hours in the absence or presence of the indicated concentrations of VPA. Cells were fixed with cold Trichloracetat (TCA) producing a final TCA concentration of 10%. After 1 hour of incubation at 4°C the cells were washed five times with water and air dried. Fixed cells were stained for 30 minutes with 0,4% (wt/vol) Sulforhodamine B (SRB) dissolved in 1% acetic acid and washed four times with 1% acetic acid to remove unbound dye. After air drying bound dye was solubilized with 10 mM unbuffered Tris base (pH 10,5) for 5 minutes. Optical densities (OD) were read on a Molecular Devices Versa Max tunable microplate reader at 520-550 nm. Four test wells for each dose-response were set in parallel with 12 control wells per cell line. Measurement of the cell population density at time 0 (T₀; the time at which the drug was added) was also made from 12 reference wells of cells fixed with TCA just prior to drug addition to the test plates. Background OD of complete medium with 5% FBS fixed and stained as described above was also determined in 12 separate wells. From the unprocessed OD data from each microtiter plate the background OD measurements (i.e. OD of complete medium plus stain and OD of cells at T₀) were subtracted thus giving the reduction of cellular biomass of the cells.

### Results:

For maximum inhibition of both, HDAC activity and cellular growth in cancer cell lines, it is important to not only achieve the required effective concentrations of VPA but also to maintain these levels as long as possible. Figure 5 shows convincingly that the degree of hyperacetylation seen in cells after treatment with VPA at concentrations above the calculated IC₅₀ values for class I HDAC enzymes is strongly enhanced when the period of exposure is prolonged. The duration of exposure was calculated as 6 hours representing the "fast release" normal VPA formulation, 15 hours representing the retarded "slow release" formulation of VPA and 24 hours representing the bi-phasic release pattern of the *PEAC* formulation.

Furthermore, results for growth inhibition by VPA obtained in two cancer cell lines, Colo320DM and PC3, indicate that VPA has to be administered for prolonged periods of time at therapeutic concentrations in order to achieve optimized growth inhibition. As can be seen in figure 6A, cell lines were exposed to 1 mM VPA for different time periods during a 72 hours culture period, ranging from 2 x 8 hours with a treatment free interval of 40 hours ("8 h d") and 2 x 20 hours with a treatment free interval of 26 hours ("20 h d") to continuous treatment of 66 hours, representative of resulting serum levels as to be achieved with the bi-phasic release principle of this invention ("continuously"). Figure 6B illustrates that growth inhibition increases with prolonged exposure to VPA. Whereas 2 x 8 hours ("8 h d") exposure leads to only 26% (PC3) and 27% (Colo320DM) growth inhibition, 2 x 20 hours ("20 h d") exposure increases growth inhibition to 43% in PC3 cells and 57% in Colo320DM cells. Maximum inhibition is seen at continuous exposure to VPA with 57% inhibition in PC3 and 80% in Colo320DM ("continuously").

Thus, the galenics needs for a most appropriate formulation of VPA for the use in cancer, autoimmune and anti-inflammatory therapy consists of a specific bi-phasic pharmacokinetic profile in order to inhibit the HDAC class I target enzymes most efficiently, to subsequently induce histone hyperacetylation in a rapid and long-lasting fashion, and to induce, e.g., maximum growth inhibition or induction of differentiation of cancer cells or other diseased hyperproliferating cells, such as immune cells in an immunological disorder. In addition, this profile may also be able to secure the efficient modulation of the desired target gene and protein expression profile which contributes to the therapeutic benefit and is suitable for the treatment or prevention of hyperproliferative, pre-malignant, and malignant diseases or autoimmune and inflammatory disorders in which the inhibition of enzymes having histone deacetylase activity has an beneficial therapeutic effect. Such disorders include but are not limited to estrogen receptor-dependent and independent breast cancer, hormone receptor-dependent and independent prostate cancer, brain cancer, renal cancer, colon and colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas as Kaposi's sarcoma and osteosarcoma, head and neck cancer, small cell and non-small cell lung carcinoma, leukemias, lymphomas and other blood cell cancers, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus and non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders (e.g. of connective tissues), atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury, obesity.

### Example 3

Manufacture of pharmaceutical compositions with the desired dissolution profil.

### 1. Manufacture of minitablets

### Formulations (weight per minitablet):

| | formulation [mg] | 1 | 2 | 3 | 4 | 5 | 6 | 7 | 8 | 9 | 10 |
|---|---|---|---|---|---|---|---|---|---|---|---|
| a | sodium valproate | 3.000 | 5.000 | 3.000 | 5.000 | 3.000 | 5.000 | 3.000 | 5.000 | 3.000 | 5.000 |
| b | calcium stearate | - | - | 0.100 | 0.167 | - | - | 0.144 | 0.240 | - | - |
| b | magnesium stearate | 0.120 | 0.200 | - | - | - | - | - | - | 0.144 | 0.240 |
| b | stearic acid | - | - | - | - | 0.150 | 0.250 | - | - | - | - |
| c | silicium dioxide | 0.111 | 0.185 | - | - | - | - | - | - | 0.105 | 0.175 |
| c | silicium dioxide, methylated | - | - | 0.120 | 0.200 | - | - | 0.111 | 0.185 | - | - |
| c | talc | - | - | - | - | 0.108 | 0.180 | - | - | - | - |
| d | ammonio methacrylate copolymer type B | - | - | 0.080 | 0.133 | - | - | 0.045 | 0.075 | - | - |
| d | ethylcellulose | - | - | - | - | 0.042 | 0.070 | - | - | - | - |
| d | Hydroxypropylmethyl cellulose | 0.069 | 0.115 | - | - | - | - | - | - | 0.051 | 0.085 |
| e | ethanol* | - | - | - | - | 0.100 | 0.167 | - | - | - | - |
| e | water* | - | - | - | - | - | - | 0.120 | 0.200 | 0.150 | 0.250 |
| | weight of minitablet | 3.300 | 5.500 | 3.300 | 5.500 | 3.300 | 5.500 | 3.300 | 5.500 | 3.300 | 5.500 |
| | diameter of minitablet [mm] | 1.7 | 2.0 | 1.7 | 2.0 | 1.7 | 2.0 | 1.7 | 2.0 | 1.7 | 2.0 |

| | | | | | | | | | | | |
|---|---|---|---|---|---|---|---|---|---|---|---|
| * no longer present in the dried finished product | | | | | | | | | | | |

### Preparation of formulations 1, 2, 3, 4 (Batch size: 1000000 minitablets):

Component "a" is mixed with 40 % of component "b", 45 % of component "c" and 60 % of component "d" in a suitable high shear mixer. The resulting mixture is then granulated on a roller compactor. The resulting granulate is blended with the residual amounts of component "b", "c" and "d" in a tumbling blender and tableted on a rotary tableting machine with the specified punch size, resulting in minitablets of the specified tablet weight.

### Preparation of formulations 5,6 (Batch size: 1000000 minitablets):

Component "a" is mixed with 55 % of component "b", 45 % of component "c" in a suitable high shear mixer. The resulting mixture is then granulated with a dispersion of "d" in "e".

The resulting granulate is dried an sieved and then blended with the residual amounts of component "b" and "c" in a tumbling blender and tableted on a rotary tableting machine with the specified punch size, resulting in minitablets of the specified tablet weight.

### Preparation of formulations 7,8,9,10 (Batch size: 1000000 minitablets):

Component "a" is mixed with 70 % of component "b", 45 % of component "c" in a suitable high shear mixer. The resulting mixture is then granulated with dispersion of "d" in "e". The resulting granulate is dried an sieved an then blended with the residual amounts of component "b" and "c" in a tumbling blender and tableted on a rotary tableting machine with the specified punch size, resulting in minitablets of the specified tablet weight.

### 2. Manufacture of slow release minitablets

### Formulations (weight per minitablet):

| | formulation [mg] | 11 | 12 | 13 | 14 | 15 | 16 | 17 | 18 | 19 | 20 |
|---|---|---|---|---|---|---|---|---|---|---|---|
| a | minitablet (1-10) | 3.300 | 3.300 | 3.300 | 3.300 | 3.300 | 5.500 | 5.500 | 5.500 | 5.500 | 5.500 |
| b | Ethylcellulose | 0.677 | - | - | 0.677 | 0.677 | 0.903 | 0.903 | - | - | 0.903 |
| b | ammonio methacrylate copolymer type B | - | 0.560 | - | - | - | - | - | 0.746 | - | - |
| b | Surelease® | - | - | 3.600 | - | - | - | - | - | 4.800 | - |
| c | Triethylcitrat | - | - | - | - | 0.144 | 0.192 | - | - | - | - |
| c | dibutyl sebacate | 0.144 | 0.110 | - | 0.144 | - | - | 0.192 | 0.147 | - | 0.192 |
| d | talc | - | 0.230 | - | 0.079 | - | - | - | 0.307 | - | 0.105 |
| d | oleic acid | 0.079 | - | - | - | 0.079 | 0.105 | 0.105 | - | - | - |
| e | ammonium hydroxid 28 %* | 0.160 | - | - | - | - | - | 0.213 | - | - | - |
| e | water* | 2.400 | 3.600 | 2.400 | 3.000 | 3.000 | 4.000 | 3.200 | 4.800 | 3.200 | 4.000 |
| | weight of coated minitablet | 4.200 | 4.200 | 4.200 | 4.200 | 4.200 | 6.700 | 6.700 | 6.700 | 6.700 | 6.700 |

| | | | | | | | | | | | |
|---|---|---|---|---|---|---|---|---|---|---|---|
| * no longer present in the dried finished product | | | | | | | | | | | |

### Preparation of formulations 11 - 20 (Batch size: 1000000 minitablets):

Component "a" is coated with a dispersion of "b", "c", "d" and "e" in a suitable coating unit.

### 3. Manufacture of fast release minitablets

### Formulations (weight per minitablet):

| | formulation [mg] | 21 | 22 | 23 | 24 | 25 | 26 | 27 | 28 |
|---|---|---|---|---|---|---|---|---|---|
| a | minitablet (1-10) | 3.300 | 3.300 | 3.300 | 3.300 | 5.500 | 5.500 | 5.500 | 5.500 |
| b | Sepifilm LP 10® | 0.250 | - | - | - | - | 0.334 | - | - |
| b | Opadry II HP® | - | 0.250 | - | - | - | - | 0.334 | - |
| b | Eudragit E PO® | - | - | 0.156 | 0.156 | 0.208 | - | - | 0.208 |
| c | stearic acid | - | - | 0.023 | 0.023 | 0.031 | - | - | 0.031 |
| c | sodium dodecyl sulfate | - | - | 0.016 | 0.016 | 0.021 | - | - | 0.021 |
| d | magnesium stearate | - | - | - | 0.055 | 0.074 | - | - | - |
| d | talc | - | - | 0.055 | - | - | - | - | 0.074 |
| e | ethanol* | - | - | - | - | - | - | - | - |
| e | water* | 2.250 | 2.250 | 1.313 | 1.313 | 1.750 | 3.000 | 3.000 | 1.750 |
| | weight of coated minitablet | 3.550 | - | 3.550 | 3.550 | 5.850 | 5.850 | - | 5.850 |

| | | | | | | | | | |
|---|---|---|---|---|---|---|---|---|---|
| * not in the dried finished product | | | | | | | | | |

### Preparation of formulations 21 - 28 (Batch size: 1000000 minitablets):

Component "a" is coated with a mixture of "b", "c", "d" and "e" in a suitable coating unit.

### 4. Manufacture of the dosage form

### Formulations (weight per dosage form):

| | Formulation [mg] | 29 | 30 | 31 | 32 | 33 | 34 | 35 | 36 | 37 | 38 |
|---|---|---|---|---|---|---|---|---|---|---|---|
| a | slow release minitablet (11-15) | 201.6 | 252.0 | 280.0 | 409.5 | 630.0 | 682.5 | 781.2 | 924.0 | 1225.0 | 1407.0 |
| b | fast release minitablet (21-24)) | 66.3 | 142.0 | 236.7 | 186.4 | 177.5 | 310.6 | 404.7 | 639.0 | 443.8 | 585.8 |
| | ratio fast slow | 1:2.6 | 1:1.5 | 1:1 | 1:1.9 | 1:3 | 1:1.9 | 1:1.6 | 1:1.2 | 1:2.3 | 1:2 |
| | sodium valproate content of dosage form | 200 | 300 | 400 | 450 | 600 | 750 | 900 | 1200 | 1250 | 1500 |
| | filling weight of dosage form | 267.9 | 394.0 | 516.7 | 595.9 | 807.5 | 993.1 | 1185.9 | 1563.0 | 1668.8 | 1992.8 |

| | Formulation [mg] | 39 | 40 | 41 | 42 | 43 | 44 | 45 | 46 | 47 | 48 |
|---|---|---|---|---|---|---|---|---|---|---|---|
| a | slow release minitablet (16-20) | 193.0 | 241.2 | 268.0 | 392.0 | 603.0 | 653.3 | 747.7 | 884.4 | 1172.5 | 1346.7 |
| b | Fast release minitablet (25-28) | 64.5 | 140.4 | 234.0 | 184.3 | 175.5 | 307.1 | 400.1 | 631.8 | 438.8 | 579.2 |
| | ratio fast: slow | 1:2.6 | 1:1.5 | 1:1 | 1:1.9 | 1:3 | 1:1.9 | 1:1.6 | 1:1.2 | 1:2.3 | 1:2 |
| | sodium valproate content of dosage form | 200 | 300 | 400 | 450 | 600 | 750 | 900 | 1200 | 1250 | 1500 |
| | filling weight of dosage form | 258.5 | 381.6 | 502.0 | 576.2 | 778.5 | 960.4 | 1147.9 | 1516.2 | 1611.3 | 1925.9 |

### Preparation of formulations 29 - 48:

Component "a" and "b" are filled in capsules or single dose containers.

Component "b" may be mixed with 0.2 % of silicium dioxide in order to reduce electrostatic phenomena.

## Claims

1. A pharmaceutical formulation comprising (i) a fast releasing component which comprises compartments containing a histone deacetylase inhibitor, and (ii) a slow releasing component which comprises compartments containing a histone deacetylase inhibitor, wherein the compartments of the fast releasing component differ from the compartments of the slow releasing component, wherein said pharmaceutical formulation exhibits a biphasic release profile, and wherein the histone deacetylase inhibitor is valproic acid or a pharmaceutically acceptable salt thereof.

2. A pharmaceutical formulation according to claim 1, wherein the histone deacetylase inhibitor is sodium valproate.

3. A pharmaceutical formulation according to claim 1 or 2 for intraveneous, intramuscular, subcutaneous, topical, oral, nasal, intraperitoneal or suppository based administration.

4. A pharmaceutical formulation according to any one of claims 1 to 3, wherein the ratio of fast releasing component to slow releasing component is between 1:0.5 and 1:4.

5. A pharmaceutical formulation according to any one of claims 1 to 4, showing an in vitro release of 20 to 50% within 30 minutes, of 25 to 65% within 2 hours, of 55 to 85% within 4 hours and 70 to 100 % within 6 hours, as determined according to USP 24, method 724, apparatus 2, in 900ml buffer pH 6.8 USP at 100 rpm.

6. A pharmaceutical formulation according to any one of claims 1 to 5, wherein the fast releasing component shows an in vitro release of at least 90% of sodium valproate within 15 minutes, and the slow releasing component shows an in vitro release of 0 to 30% within 1 hour, of 20 to 60% within 4 hours and of 55 to 95% within 6 hours, as determined according to USP 24, method 724, apparatus 2, in 900ml buffer pH 6.8 USP at 100 rpm.

7. A pharmaceutical formulation according to any one of claims 1 to 6, wherein the slow releasing components have a content of histone deacetylase inhibitor of 50 to 96% by weight, and the fast releasing components have a content of histone deacetylase inhibitor of 50 to 96% by weight.

8. A pharmaceutical formulation according to any one of claims 1 to 7, being a multiple unit dosage form comprising compartments, wherein the maximum size of the single compartments is 3 mm.

9. A pharmaceutical formulation according to claim 8, wherein the size of the single compartments is 0.5 - 2.5 mm.

10. A pharmaceutical formulation according to any one of claims 1 to 9, comprising 0.1 to 3g of histone deacetylase inhibitor.

11. A pharmaceutical formulation according to any one of claims 1 to 10, wherein the fast releasing component comprises coated minitablets, and the slow releasing component comprises coated minitablets.

12. A pharmaceutical formulation according to claim 11 wherein the coated minitablets comprise sodium valproate, a lubricant, a polymer and a glidant.

13. A pharmaceutical formulation according to claim 12 wherein the lubricant is magnesium stearate, calcium stearate or stearic acid.

14. A pharmaceutical formulation according to claim 12 or 13, wherein the glidant is silicium dioxide, methylated silicium dioxide or talc.

15. A pharmaceutical formulation according to any one of claims 12 to 14, wherein the polymer is ammonio methacrylate copolymer, ethylcellulose or hypromellose.

16. A pharmaceutical formulation according to any one of claims 11 to 15, wherein the coating of the coated minitablets of the fast release component comprises at least one polymer and at least one suitable plasticizer.

17. A pharmaceutical formulation according to claim 16 wherein the polymer is aminoalkyl methacrylate copolymer, polyvinyl alcohol or hypromellose.

18. A pharmaceutical formulation according to any one of claims 11 to 17, wherein the coating of the coated minitablets of the slow release component comprises at least a polymer and at least one suitable plasticizer.

19. A pharmaceutical formulation according to claim 18 wherein the polymer is ammonio methacrylate copolymer or ethylcellulose.

20. A pharmaceutical formulation according to any one of claims 1 to 19, which is an orally available pharmaceutical formulation.

21. A pharmaceutical formulation according to any one of claims 1 to 20, wherein the release profile is determined according to USP 24, method 724, apparatus 2, in 900ml buffer pH 6.8 USP at 100 rpm.

22. The use of a pharmaceutical formulation according to any one of the preceding claims for the manufacture of a medicament for the treatment or prevention of estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemias, lymphomas and/or other blood cell cancers, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity.

23. The use of valproic acid or a pharmaceutically acceptable salt thereof for the manufacture of a medicament for the treatment or prevention of estrogen receptor-dependent breast cancer, estrogen receptor-independent breast cancer, hormone receptor-dependent prostate cancer, hormone receptor-independent prostate cancer, brain cancer, renal cancer, colon cancer, colorectal cancer, pancreatic cancer, bladder cancer, esophageal cancer, stomach cancer, genitourinary cancer, gastrointestinal cancer, uterine cancer, ovarian cancer, astrocytomas, gliomas, skin cancer, squamous cell carcinoma, Keratoakantoma, Bowen disease, cutaneous T-Cell Lymphoma, melanoma, basal cell carcinoma, actinic keratosis; ichtiosis; acne, acne vulgaris, sarcomas, Kaposi's sarcoma, osteosarcoma, head and neck cancer, small cell lung carcinoma, non-small cell lung carcinoma, leukemias, lymphomas and/or other blood cell cancers, thyroid resistance syndrome, diabetes, thalassemia, cirrhosis, protozoal infection, rheumatoid arthritis, rheumatoid spondylitis, all forms of rheumatism, osteoarthritis, gouty arthritis, multiple sclerosis, insulin dependent diabetes mellitus, non-insulin dependent diabetes, asthma, rhinitis, uveithis, lupus erythematoidis, ulcerative colitis, Morbus Crohn, inflammatory bowel disease, chronic diarrhea, psoriasis, atopic dermatitis, bone disease, fibroproliferative disorders, atherosclerosis, aplastic anemia, DiGeorge syndrome, Graves' disease, epilepsia, status epilepticus, alzheimer's disease, depression, schizophrenia, schizoaffective disorder, mania, stroke, mood-incongruent psychotic symptoms, bipolar disorder, affective disorders, meningitis, muscular dystrophy, multiple sclerosis, agitation, cardiac hypertrophy, heart failure, reperfusion injury and/or obesity, wherein the medicament is a pharmaceutical formulation according to any one of claims 1 to 21.

24. The use according to claim 22 or 23, wherein the medicament is administered through intraveneous, intramuscular, subcutaneous, topical, oral, nasal, intraperitoneal or suppository based application.

25. A method for the preparation of a pharmaceutical formulation according to claim 1, comprising combining a fast releasing component containing a histone deacetylase inhibitor with a slow releasing component containing a histone deacetylase inhibitor such that a multiple unit dosage form comprising compartments is obtained, wherein the histone deacetylase inhibitor is valproic acid or a pharmaceutically acceptable salt thereof.

26. A method according to claim 25, wherein the single drug containing compartments are prepared by granulation, extrusion, hot melt, pelletizing, tabletting and coating techniques.

27. A method according to claim 25 or 26, wherein the fast releasing components and the slow releasing component are mixed in a predefined proportion and filled in capsules or containers for single dose administration.

28. A method according to claim 25 or 26, wherein the fast and the slow releasing components are filled successively in capsules or containers for single dose administration without mixing them beforehand.

## Patentansprüche

1. Pharmazeutische Formulierung, umfassend (i) eine Komponente mit schneller Freisetzung, die Kompartimente umfaßt, die einen Histondeacetylaseinhibitor enthalten, und (ii) eine Komponente mit langsamer Freisetzung, die Kompartimente umfaßt, die einen Histondeacetylaseinhibitor enthalten, wobei sich die Kompartimente der Komponente mit schneller Freisetzung von den Kompartimenten der Komponente mit langsamer Freisetzung unterscheiden, wobei die pharmazeutische Formulierung ein zweiphasiges Freisetzungsprofil aufweist, und wobei der Histondeacetylaseinhibitor Valproinäure oder ein pharmazeutisch akzeptables Salz davon ist.

2. Pharmazeutische Formulierung nach Anspruch 1, wobei der Histondeacetylaseinhibitor Natriumvalproat ist.

3. Pharmazeutische Formulierung nach Anspruch 1 oder 2 für intravenöse, intramuskuläre, subkutane, topische, orale, nasale, intraperitoneale oder Zäpfchenverabreichung.

4. Pharmazeutische Formulierung nach einem der Ansprüche 1 bis 3, wobei das Verhältnis der Komponente mit schneller Freisetzung zu der Komponente mit langsamer Freisetzung zwischen 1 : 0,5 und 1 : 4 liegt.

5. Pharmazeutische Formulierung nach einem der Ansprüche 1 bis 4, die eine In-vitro-Freisetzung von 20 bis 50 % innerhalb von 30 Minuten, von 25 bis 65 % innerhalb von 2 Stunden, von 55 bis 85 % innerhalb von 4 Stunden und von 70 bis 100 % innerhalb von 6 Stunden, bestimmt gemäß USP 24, Verfahren 724, Apparat 2, in 900 ml Puffer, pH 6,8 USP, bei 100 U/min, zeigt.

6. Pharmazeutische Formulierung nach einem der Ansprüche 1 bis 5, wobei die Komponente mit schneller Freisetzung eine In-vitro-Freisetzung von mindestens 90 % Natriumvalproat innerhalb von 15 Minuten zeigt, und die Komponente mit langsamer Freisetzung eine In-vitro-Freisetzung von 0 bis 30 % innerhalb von 1 Stunde, von 20 bis 60 % innerhalb von 4 Stunden und von 55 bis 95 % innerhalb von 6 Stunden, bestimmt gemäß USP 24, Verfahren 724, Apparat 2, in 900 ml Puffer, pH 6,8 USP, bei 100 U/min, zeigt.

7. Pharmazeutische Formulierung nach einem der Ansprüche 1 bis 6, wobei die Komponenten mit langsamer Freisetzung einen Gehalt des Histondeacetylaseinhibitors von 50 bis 96 Gew.-% aufweisen und die Komponenten mit schneller Freisetzung einen Gehalt des Histondeacetylaseinhibitors von 50 bis 96 Gew.-% aufweisen.

8. Pharmazeutische Formulierung nach einem der Ansprüche 1 bis 7, die eine Mehrfacheinheitsdosisform, umfassend Kompartimente, wobei die maximale Größe der einzelnen Kompartimente 3 mm beträgt, ist.

9. Pharmazeutische Formulierung nach Anspruch 8, wobei die Größe der einzelnen Kompartimente 0,5 - 2,5 mm beträgt.

10. Pharmazeutische Formulierung nach einem der Ansprüche 1 bis 9, umfassend 0,1 bis 3 g Histondeacetylaseinhibitor.

11. Pharmazeutische Formulierung nach einem der Ansprüche 1 bis 10, wobei die Komponente mit schneller Freisetzung beschichtete Minitabletten umfaßt und die Komponente mit langsamer Freisetzung beschichtete Minitabletten umfaßt.

12. Pharmazeutische Formulierung nach Anspruch 11, wobei die beschichteten Minitabletten Natriumvalproat, ein Schmiermittel, ein Polymer und ein Gleitmittel umfassen.

13. Pharmazeutische Formulierung nach Anspruch 12, wobei das Schmiermittel Magnesiumstearat, Calciumstearat oder Stearinsäure ist.

14. Pharmazeutische Formulierung nach Anspruch 12 oder 13, wobei das Gleitmittel Siliciumdioxid, methyliertes Siliciumdioxid oder Talk ist.

15. Pharmazeutische Formulierung nach einem der Ansprüche 12 bis 14, wobei das Polymer Ammoniummethacrylatcopolymer, Ethylcellulose oder Hypromellose.

16. Pharmazeutische Formulierung nach einem der Ansprüche 11 bis 15, wobei die Beschichtung der beschichteten Minitabletten der Komponente mit schneller Freisetzung mindestens ein Polymer und mindestens einen geeigneten Weichmacher umfaßt.

17. Pharmazeutische Formulierung nach Anspruch 16, wobei das Polymer Aminoalkylmethacrylatcopolymer, Polyvinylalkohol oder Hypromellose ist.

18. Pharmazeutische Formulierung nach einem der Ansprüche 11 bis 17, wobei die Beschichtung der beschichteten Minitabletten der Komponente mit langsamer Freisetzung mindestens ein Polymer und mindestens einen geeigneten Weichmacher umfaßt.

19. Pharmazeutische Formulierung nach Anspruch 18, wobei das Polymer Ammoniummethacrylatcopolymer oder Ethylcellulose ist.

20. Pharmazeutische Formulierung nach einem der Ansprüche 1 bis 19, die eine oral verfügbare pharmazeutische Formulierung ist.

21. Pharmazeutische Formulierung nach einem der Ansprüche 1 bis 20, wobei das Freisetzungsprofil gemäß USP 24, Verfahren 724, Apparat 2, in 900 ml Puffer, pH 6,8 USP, bei 100 U/min bestimmt wird.

22. Verwendung einer pharmazeutischen Formulierung nach einem der vorhergehenden Ansprüche zur Herstellung eines Medikaments für die Behandlung oder Vorbeugung von Östrogenrezeptor-abhängigem Brustkrebs, Östrogenrezeptor-unabhängigem Brustkrebs, Hormonrezeptor-abhängigem Prostatakrebs, Hormonrezeptor-unabhängigem Prostatakrebs, Gehirnkrebs, Nierenkrebs, Kolonkrebs, kolorektalem Karzinom, Pankreaskarzinom, Blasenkarzinom, Ösophaguskarzinom, Magenkrebs, Urogenitalkrebs, Magendarmkrebs, Gebärmutterkrebs, Eierstockkrebs, Astrozytomen, Gliomen, Hautkrebs, Plattenepithelkarzinom, Keratoakanthomen, Bowen-Krankheit, kutanem T-Zell-Lymphom, Melanom, Basalzellkarzinom, Strahlenkeratose; Ichthyose; Akne, Akne vulgaris, Sarkomen, Kaposi-Sarkom, Osteosarkom, Kopf- und Halskrebs, kleinzelligem Bronchialkarzinom, nicht kleinzelligem Bronchialkarzinom, Leukämien, Lymphomen und/oder anderen Blutzellenkrebsen, Schilddrüsenresistenz-Syndrom, Diabetes, Thalassämie, Zirrhose, Protozoeninfektion, Rheumatoidarthritis, rheumatoider Spondylitis, allen Formen von Rheumatismus, Osteoarthritis, Gichtarthritis, multipler Sklerose, insulinpflichtigem Diabetes mellitus, nicht insulinpflichtigem Diabetes, Asthma, Rhinitis, Uveitis, Lupus erythematodes, Colitis ulcerosa, Crohn-Krankheit, entzündlicher Darmerkrankung, chronischer Diarrhöe, Psoriasis, atopischer Dermatitis, Knochenerkrankung, fibroproliferativen Störungen, Atherosklerose, aplastischer Anämie, DiGeorge-Syndrom, Basedow-Krankheit, Epilepsie, Status epilepticus, Alzheimer-Krankheit, Depression, Schizophrenie, schizoaffektiver Störung, Manie, Schlaganfall, stimmungsinkongruenten psychotischen Symptomen, bipolarer Störung, Affektivstörungen, Meningitis, Muskeldystrophie, multipler Sklerose, Agitation, Herzhypertrophie, Herzinsuffizienz, Reperfusionsverletzung und/oder Fettleibig.

23. Verwendung von Valproinsäure oder eines pharmazeutisch akzeptablen Salzes davon zur Herstellung eines Medikaments für die Behandlung oder Vorbeugung von Östrogenrezeptorabhängigem Brustkrebs, Östrogenrezeptor-unabhängigem Brustkrebs, Hormonrezeptorabhängigem Prostatakrebs, Hormonrezeptor-unabhängigem Prostatakrebs, Gehirnkrebs, Nierenkrebs, Kolonkrebs, kolorektalem Karzinom, Pankreaskarzinom, Blasenkarzinom, Ösophaguskarzinom, Magenkrebs, Urogenitalkrebs, Magendarmkrebs, Gebärmutterkrebs, Eierstockkrebs, Astrozytomen, Gliomen, Hautkrebs, Plattenepithelkarzinom, Keratoakanthomen, Bowen-Krankheit, kutanem T-Zell-Lymphom, Melanom, Basalzellkarzinom, Strahlenkeratose; Ichthyose; Akne, Akne vulgaris, Sarkomen, Kaposi-Sarkom, Osteosarkom, Kopf- und Halskrebs, kleinzelligem Bronchialkarzinom, nicht kleinzelligem Bronchialkarzinom, Leukämien, Lymphomen und/oder anderen Blutzellenkrebsen, Schilddrüsenresistenz-Syndrom, Diabetes, Thalassämie, Zirrhose, Protozoeninfektion, Rheumatoidarthritis, rheumatoider Spondylitis, allen Formen von Rheumatismus, Osteoarthritis, Gichtarthritis, multipler Sklerose, insulinpflichtigem Diabetes mellitus, nicht insulinpflichtigem Diabetes, Asthma, Rhinitis, Uveitis, Lupus erythematodes, Colitis ulcerosa, Crohn-Krankheit, entzündlicher Darmerkrankung, chronischer Diarrhöe, Psoriasis, atopischer Dermatitis, Knochenerkrankung, fibroproliferativen Störungen, Atherosklerose, aplastischer Anämie, DiGeorge-Syndrom, Basedow-Krankheit, Epilepsie, Status epilepticus, Alzheimer-Krankheit, Depression, Schizophrenie, schizoaffektiver Störung, Manie, Schlaganfall, stimmungsinkongruenten psychotischen Symptomen, bipolarer Störung, Affektivstörungen, Meningitis, Muskeldystrophie, multipler Sklerose, Agitation, Herzhypertrophie, Herzinsuffizienz, Reperfusionsverletzung und/oder Fettleibig, wobei das Medikament eine pharmazeutische Formulierung nach einem der Ansprüche 1 bis 21 ist.

24. Verwendung nach Anspruch 22 oder 23, wobei das Medikament durch intravenöse, intramuskuläre, subkutane, topische, orale, nasale, intraperitoneale Applikation oder als Zäpfchen verabreicht wird.

25. Verfahren zur Herstellung einer pharmazeutischen Formulierung nach Anspruch 1, umfassend das Kombinieren einer Komponente mit schneller Freisetzung, die einen Histondeacetylaseinhibitor enthält, mit einer Komponente mit langsamer Freisetzung, die einen Histondeacetylaseinhibitor enthält, so daß eine Mehrfacheinheitsdosisform, umfassend Kompartimente, erhalten wird, wobei der Histondeacetylaseinhibitor Valproinsäure oder ein pharmazeutisch akzeptables Salz davon ist.

26. Verfahren nach Anspruch 25, wobei die Kompartimente, die das einzelne Arzneimittel enthalten, durch Granulations-, Extrusions-, Heißschmelz-, Pelletisier-, Tablettier- und Beschichtungstechniken hergestellt werden.

27. Verfahren nach Anspruch 25 oder 26, wobei die Komponenten mit schneller Freisetzung und die Komponente mit langsamer Freisetzung in einem vorher bestimmten Anteil gemischt und in Kapseln oder Behälter für eine Einzeldosisverabreichung gefüllt werden.

28. Verfahren nach Anspruch 25 oder 26, wobei die Komponente mit schneller und die Komponente mit langsamer Freisetzung nacheinander in Kapseln oder Behälter für eine Einzeldosisverabreichung gefüllt werden, ohne daß sie zuvor gemischt wurden.

## Revendications

1. Formulation pharmaceutique comprenant (i) un composant à libération rapide qui comprend des compartiments contenant un inhibiteur d'histone désacétylase, et (ii) un composant à libération lente qui comprend des compartiments contenant un inhibiteur d'histone désacétylase, dans laquelle les compartiments du composant à libération rapide diffèrent des compartiments du composant à libération lente, dans laquelle ladite formulation pharmaceutique montre un profil de libération biphasique, et dans laquelle l'inhibiteur d'histone désacétylase est de l'acide valproïque ou un sel pharmaceutiquement acceptable de celui-ci.

2. Formulation pharmaceutique suivant la revendication 1, dans laquelle l'inhibiteur d'histone désacétylase est du valproate de sodium.

3. Formulation pharmaceutique suivant l'une ou l'autre des revendications 1 et 2, pour une administration intraveineuse, intramusculaire, sous-cutanée, topique, orale, nasale, intrapéritonéale ou à base de suppositoire.

4. Formulation pharmaceutique suivant l'une quelconque des revendications 1 à 3, dans laquelle le rapport de composant à libération rapide au composant à libération lente se situe entre 1/0,5 et 1/4.

5. Formulation pharmaceutique suivant l'une quelconque des revendications 1 à 4, montrant une libération in vitro de 20 à 50 % après 30 minutes, de 25 à 65 % après 2 heures, de 55 à 85 % après 4 heures et de 70 à 100 % après 6 heures, telles que déterminées suivant l'USP 24, méthode 724, appareil 2, dans un tampon de 900 ml de pH 6,8 USP à 100 tpm.

6. Formulation pharmaceutique suivant l'une quelconque des revendications 1 à 5, dans laquelle le composant à libération rapide montre une libération in vitro d'au moins 90 % de valproate de sodium après 15 minutes, et le composant à libération lente montre une libération in vitro de 0 à 30 % après 1 heure, de 20 à 60 % après 4 heures et de 55 à 95 % après 6 heures, telles que déterminées suivant l'USP 24, méthode 724, appareil 2, dans un tampon de 900 ml de pH 6,8 USP à 100 tpm.

7. Formulation pharmaceutique suivant l'une quelconque des revendications 1 à 6, dans laquelle les composants à libération lente ont une teneur en inhibiteur d'histone désacétylase de 50 à 96 % en poids, et les composants à libération rapide ont une teneur en inhibiteur d'histone désacétylase de 50 à 96 % en poids.

8. Formulation pharmaceutique suivant l'une quelconque des revendications 1 à 7, constituant une forme posologique unitaire multiple comprenant des compartiments, dans laquelle la taille maximale des compartiments isolés est de 3 mm.

9. Formulation pharmaceutique suivant la revendication 8, dans laquelle la taille des compartiments isolés est de 0,5-2,5 mm.

10. Formulation pharmaceutique suivant l'une quelconque des revendications 1 à 9, comprenant 0,1 à 3 g d'inhibiteur d'histone désacétylase.

11. Formulation pharmaceutique suivant l'une quelconque des revendications 1 à 10, dans laquelle le composant à libération rapide comprend des minicomprimés enrobés, et le composant à libération lente comprend des minicomprimés enrobés.

12. Formulation pharmaceutique suivant la revendication 11, dans laquelle les minicomprimés enrobés comprennent du valproate de sodium, un lubrifiant, un polymère et un agent de glissement.

13. Formulation pharmaceutique suivant la revendication 12, dans laquelle le lubrifiant est du stéarate de magnésium, du stéarate de calcium ou de l'acide stéarique.

14. Formulation pharmaceutique suivant l'une ou l'autre des revendications 12 et 13, dans laquelle l'agent de glissement est du dioxyde de silicium, du dioxyde de silicium méthylé ou du talc.

15. Formulation pharmaceutique suivant l'une quelconque des revendications 12 à 14, dans laquelle le polymère est un copolymère d'ammoniométhacrylate, de l'éthylcellulose ou de l'hypromellose.

16. Formulation pharmaceutique suivant l'une quelconque des revendications 11 à 15, dans laquelle l'enrobage des minicomprimés enrobés du composant à libération rapide comprend au moins un polymère et au moins un plastifiant approprié.

17. Formulation pharmaceutique suivant la revendication 16, dans laquelle le polymère est un copolymère de méthacrylate d'aminoalkyle, de l'alcool polyvinylique ou de l'hypromellose.

18. Formulation pharmaceutique suivant l'une quelconque des revendications 11 à 17, dans laquelle l'enrobage des minicomprimés enrobés du composant à libération lente comprend au moins un polymère et au moins un plastifiant approprié.

19. Formulation pharmaceutique suivant la revendication 18, dans laquelle le polymère est un copolymère d'ammoniométhacrylate ou de l'éthylcellulose.

20. Formulation pharmaceutique suivant l'une quelconque des revendications 1 à 19, qui est une formulation pharmaceutique disponible oralement.

21. Formulation pharmaceutique suivant l'une quelconque des revendications 1 à 20, dans laquelle le profil de libération est déterminé suivant l'USP 24, méthode 724, appareil 2, dans un tampon de 900 ml de pH 6,8 USP à 100 tpm.

22. Utilisation d'une formulation pharmaceutique suivant l'une quelconque des revendications précédentes pour la fabrication d'un médicament pour le traitement ou la prévention du cancer du sein dépendant des récepteurs des oestrogènes, du cancer du sein indépendant des récepteurs des oestrogènes, du cancer de la prostate dépendant des récepteurs hormonaux, du cancer de la prostate indépendant des récepteurs hormonaux, du cancer du cerveau, du cancer rénal, du cancer du côlon, du cancer colorectal, du cancer pancréatique, du cancer de la vessie, du cancer oesophagien, du cancer de l'estomac, du cancer génito-urinaire, du cancer gastro-intestinal, du cancer utérin, du cancer ovarien, des astrocytomes, des gliomes, du cancer de la peau, de l'épithélioma épidermoïde, du kérato-acanthome, de la maladie de Bowen, du lymphome T cutané, du mélanome, du carcinome basocellulaire, de la kératose sénile, de l'ichtyose, de l'acné, de l'acné vulgaire, des sarcomes, du sarcome de Kaposi, de l'ostéosarcome, du cancer de la tête et du cou, du cancer pulmonaire à petites cellules, du cancer bronchique non à petites cellules, des leucémies, des lymphomes et/ou autres cancers des globules sanguins, du syndrome de la résistance thyroïdienne, des diabètes, de la thalassémie, de la cirrhose, d'une infection protozoaire, de la polyarthrite rhumatoïde, de la spondylarthrite ankylosante, de toutes les formes de rhumatisme, de l'ostéoarthrite, de l'arthrite goutteuse, de la sclérose multiple, du diabète insulinodépendant, du diabète non insulinodépendant, de l'asthme, de la rhinite, du lupus érythémateux, de la colite ulcérative, de la maladie de Crohn, des maladies inflammatoires chroniques de l'intestin, de la diarrhée chronique, du psoriasis, de la dermatite atopique, des maladies osseuses, des affections fibroprolifératives, de l'athérosclérose, de l'anémie aplastique, du syndrome de DiGeorge, de la maladie de Graves, de l'épilepsie, de l'état de mal épileptique, de la maladie d'Alzheimer, de la dépression, de la schizophrénie, des troubles schizoaffectifs, de la manie, des attaques, des symptômes psychotiques non concluant de l'humeur, des troubles bipolaires, des troubles affectifs, de la méningite, de la dystrophie musculaire, de la sclérose multiple, de l'agitation, de l'hypertrophie cardiaque, de l'insuffisance cardiaque, des lésions de reperfusion et/ou de l'obésité.

23. Utilisation d'acide valproïque ou d'un sel pharmaceutiquement acceptable de celui-ci pour la fabrication d'un médicament pour le traitement ou la prévention du cancer du sein dépendant des récepteurs des oestrogènes, du cancer du sein indépendant des récepteurs des oestrogènes, du cancer de la prostate dépendant des récepteurs hormonaux, du cancer de la prostate indépendant des récepteurs hormonaux, du cancer du cerveau, du cancer rénal, du cancer du côlon, du cancer colorectal, du cancer pancréatique, du cancer de la vessie, du cancer oesophagien, du cancer de l'estomac, du cancer génito-urinaire, du cancer gastro-intestinal, du cancer utérin, du cancer ovarien, des astrocytomes, des gliomes, du cancer de la peau, de l'épithélioma épidermoïde, du kérato-acanthome, de la maladie de Bowen, du lymphome T cutané, du mélanome, du carcinome basocellulaire, de la kératose sénile, de l'ichtyose, de l'acné, de l'acné vulgaire, des sarcomes, du sarcome de Kaposi, de l'ostéosarcome, du cancer de la tête et du cou, du cancer pulmonaire à petites cellules, du cancer bronchique non à petites cellules, des leucémies, des lymphomes et/ou autres cancers des globules sanguins, du syndrome de la résistance thyroïdienne, des diabètes, de la thalassémie, de la cirrhose, d'une infection protozoaire, de la polyarthrite rhumatoïde, de la spondylarthrite ankylosante, de toutes les formes de rhumatisme, de l'ostéoarthrite, de l'arthrite goutteuse, de la sclérose multiple, du diabète insulinodépendant, du diabète non insulinodépendant, de l'asthme, de la rhinite, du lupus érythémateux, de la colite ulcérative, de la maladie de Crohn, des maladies inflammatoires chroniques de l'intestin, de la diarrhée chronique, du psoriasis, de la dermatite atopique, des maladies osseuses, des affections fibroprolifératives, de l'athérosclérose, de l'anémie aplastique, du syndrome de DiGeorge, de la maladie de Graves, de l'épilepsie, de l'état de mal épileptique, de la maladie d'Alzheimer, de la dépression, de la schizophrénie, des troubles schizoaffectifs, de la manie, des attaques, des symptômes psychotiques non concluant de l'humeur, des troubles bipolaires, des troubles affectifs, de la méningite, de la dystrophie musculaire, de la sclérose multiple, de l'agitation, de l'hypertrophie cardiaque, de l'insuffisance cardiaque, des lésions de reperfusion et/ou de l'obésité, dans laquelle le médicament est une formulation pharmaceutique suivant l'une quelconque des revendications 1 à 21.

24. Utilisation suivant l'une ou l'autre des revendications 22 et 23, dans laquelle le médicament est administré par application intraveineuse, intramusculaire, sous-cutanée, topique, orale, nasale, intrapéritonéale ou par suppositoire.

25. Procédé de préparation d'une formulation pharmaceutique suivant la revendication 1, comprenant la combinaison d'un composant à libération rapide contenant un inhibiteur d'histone désacétylase avec un composant à libération lente contenant un inhibiteur d'histone désacétylase de telle sorte qu'une forme posologique unitaire multiple comprenant des compartiments soit obtenue, dans lequel l'inhibiteur d'histone désacétylase est de l'acide valproïque ou un sel pharmaceutiquement acceptable de celui-ci.

26. Procédé suivant la revendication 25, dans lequel les compartiments pris isolément contenant du médicament sont préparés par des techniques de granulation, d'extrusion, de fusion à chaud, de regranulation, de fabrication de comprimés et d'enrobage.

27. Procédé suivant l'une ou l'autre des revendications 25 et 26, dans lequel les composants à libération rapide et le composant à libération lente sont mélangés dans une proportion prédéfinie et versés dans des capsules ou des récipients pour une administration à dose unique.

28. Procédé suivant l'une ou l'autre des revendications 25 et 26, dans lequel les composants à libération rapide et lente sont versés successivement dans des capsules ou des récipients pour une administration à dose unique sans les mélanger préalablement.
